# EUROPEAN PATENT SPECIFICATION

(11) **EP 0 440 350 B1**
(45) Date of publication and mention of the grant of the patent: **27.08.1997**
(21) Application number: 91300345.5
(22) Date of filing: 17.01.1991
(51) Int. Cl.: G01N 33/543, A61B 5/117, G01N 33/92, G01N 33/94, C12Q 1/28, G01N 33/52

(54) **Blood testing and fingerprint identification method and device**
Gerät und Verfahren zur Untersuchung von Blut und zur Fingerabdruck-Identifikation
Dispositif et procédé d'analyse du sang et identification d'empreintes digitales

(30) Priority: 19.01.1990 US 467532
(43) Date of publication of application: 07.08.1991
(73) Proprietor: LA MINA LTD., Rockville, Maryland 20852 (US)
(72) Inventor: Guirguis, Raouf A., Rockville, Maryland 20852 (US)
(74) Representative: McCall, John Douglas

(56) References cited:
- EP-A- 0 203 238
- EP-A- 0 233 063
- EP-A- 0 279 213
- WO-A-86/00712
- US-A- 3 419 000
- US-A- 4 029 012
- WORLD PATENTS INDEX LATEST Week 8440, Derwent Publications Ltd., London, GB; AN 84-245696

## Description

The present invention is directed to an individual testing method and device and more specifically to a method and device for detecting the presence of specific antigens or specific antibodies produced by drugs, in a biological fluid such as blood and using the device to also positively identify the individual tested by reproducing the fingerprint of the person being tested. Previously drug testing has been accomplished by testing individual fluid samples such as urine or blood to determine the presence of drugs in the body. Such testing procedures are very common in the athletic world, prisons, courts of law, and in the general workplace and are many times proscribed by contracts between the individual and his/her employer or labor union which represents the individual or group. A problem which has occurred during such testing is that test fluids are obtained from persons other than the person to be tested or that test fluids become mixed, lost, or cannot be specifically identified with that person after the test comes back with positive results.

The present invention attempts to overcome the problems which are inherent in the prior art through the use of a specifically designed fingerprint pad device which tests for the presence of drugs or other specified agents in the blood as well as providing a fingerprint of the person giving the test so that positive identification of the fluid donor is irrefutably obtained.

The family of immunoassays works upon the single principle that is the specific recognition of an antigen by an antibody. The specific antigen detection and quantification requires an antibody which recognizes the uniqueness of an antigen. One unique binding site serves as an identifying marker for that protein.

Antibodies, immobilized (irreversibly bound) on the membrane is well known in the art and such antibodies are designed to have binding sites which have high affinity for the epitopes of the antigens carried in the blood and vice versa. Covalent binding of protein to the membrane surface offers a permanent bond which is irreversible, so that once a protein like an antibody is bound, it will not be desorbed during an assay. The principle of affinity chromatography requires that a successful separation of a biospecific ligand is available and that it can be chemically immobilized to a chromatographic bed material, the matrix. Numbers of methods well known in the art have been used to couple or immobilize the antigen to a variety of activated resins. Examples of immobilization techniques which exhibit variable linkage are those formed by the reaction of the reactive groups on the support with amino, thiol, hydroxyl, and carboxyl groups on the protein ligand. The selection of the ligand is influenced by two factors. First, the ligand should exhibit specific and reversible binding affinity for the substance to be purified and secondly it should have chemically modifiable groups which allow it to be attached to the matrix without destroying its binding activity. (Examples of such are Protein G manufactured by Pharmacia, Hydrazide AvidGel Ax manufactured by BioProbe International, and Actigel-ALD manufactured by Sterogene Bioseparation Inc.)

When a definitive antibody for a given antigen is available, it is used to identify the antigen in the sample mixture. Once the antibody combines with the antigen, a means is needed to recognize the complex. This has been accomplished in the past by providing a labelled antibody, such as an enzyme, enzyme link immunosorbent (ELISA)-type assay so that the site is incubated with a chromogenic substrate and a color is developed whose intensity is proportional to the enzyme label present.

It is known in the prior art to use membranes in immunoassay testing and also to use such membranes in connection with an absorbent pad to permit the creation of a self-contained package, which is easily disposable.(American Clinical Products Review, June 1987) Such membrane structures have been developed by Millipore Corp. and other manufacturers. Various problems have occurred with the use of such membranes which are primarily used in strip testing due to different plastics which have been used and the degradation for absorption of the protein from the affinity membrane.

EP-A- 0203238 discloses a device and method for the determination of ligands in body fluids.

JP-A- 51104028 discloses a method in which a fingerprint (which may have blood deposited thereon) is transferred to a film and stuck onto a glass slide for testing.

It is therefore desirable to provide an easy to handle disposable testing pad which holds a blood sample taken from a finger puncture on a membrane substrate having a specific immobilized antibody or antigen bed to capture a concentrated amount of specified antigen or antibody from the blood while simultaneously providing the fingerprint indentation of the user to be recorded for positive identification.

The invention is directed toward a blood antigen collection device for testing and identification. The device is in the form of an absorbent base having a permeable membrane mounted thereon which is coated with specific antibodies. The membrane bed antibodies capture specific antigens carried by the blood to determine the presence in the body of specific drugs. It is, of course, apparent that antigens and antibodies can be switched and either immobilized to capture the other.

It is an object of the invention to collect antigen and/or antibodies from blood samples removed from the body for testing and simultaneously use the fingerprint pattern to positively identify the donor of the sample. Previously such testing has been accomplished by a series of tests which may involve shifting of the fluid being tested to different containers and removal of the fluid from the person being tested to a place distant from the person of the individual which allowed fluid misplacement and substitution and questions as to the chain of title of the tested fluid.

According to the present invention there is provided a test device, for testing for the presence of at least one substance in a fluid sample obtained from a human being whilst simultaneously positively identifying the sample as coming from the test subject, comprising a porous or permeable membrane mounted to or over an absorbent pad, said porous or permeable membrane comprising at least one immobilized first antibody or antigen to capture a concentrated amount of at least one antigen or antibody specific to said at least one first antigen or antibody from the fluid sample in direct proportion to the concentration of the at least one antigen or antibody in the fluid sample, said porous or permeable membrane being simultaneously capable of retaining a fingerprint imprint from the test subject's finger.

According to a further aspect of the present invention there is provided a method, for testing for a substance in a fluid sample obtained from a human being whilst simultaneously positively identifying the sample as coming from the test subject, comprising the steps of:
a) obtaining a fluid sample from the test subject;
b) covering the finger with a thin film of the fluid sample;
c) placing the finger on a porous or permeable membrane having an absorbent pad mounted thereto or behind thereof so that the fluid is deposited from the finger onto the porous or permeable membrane, said porous or permeable membrane comprising at least one immobilised first antibody or antigen to capture a concentrated amount of at least one antigen or antibody specific to said first antigen or antibody from the fluid sample in direct proportion to the concentration of the at least one antigen or antibody specific to said first antigen or antibody in the fluid sample; and
d) adding a colour development solution to the porous or permeable membrane to identify the presence or absence of said at least one substance and identify the test subject's fingerprint.

In the accompanying drawings there is shown as illustrated embodiment of the invention from which these and other objectives, novel features and advantages will be readily apparent. In the drawings:-
Figure 1 is a schematic view of a finger being punctured;
Figure 2 is a schematic view of a spatula spreading the blood droplet taken from the punctured finger;
Figure 3 is an elevational view of the finger placed and held on the inventive testing pad device;
Figure 4 is a top plan view of the architecture of the fingerprint;
Figure 5 is a cross-sectional representation of the architecture of the fingerprint shown in Figure 4;
Figure 6 is a schematic view of the finger covered with a thin film of blood before touching the testing pad;
Figure 7 is a schematic cross-sectional view of the blood placed by the finger on the reaction membrane;
Figure 8 is a side view of color development solution being placed on the pad with fingerprint; and
Figure 9 is a top plan view of the fingerprint testing pad device showing the developed fingerprint with a positive indication of drugs in the bloodstream.

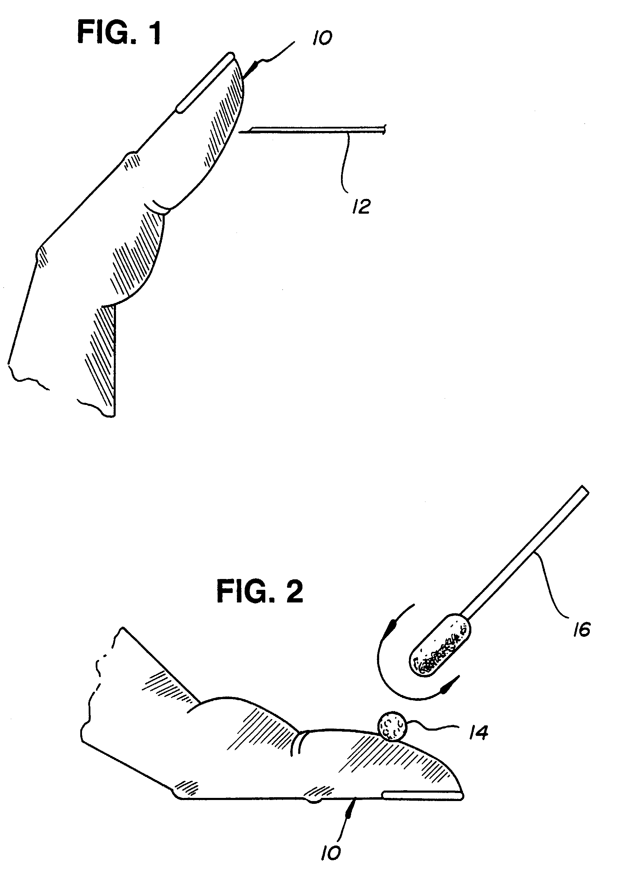

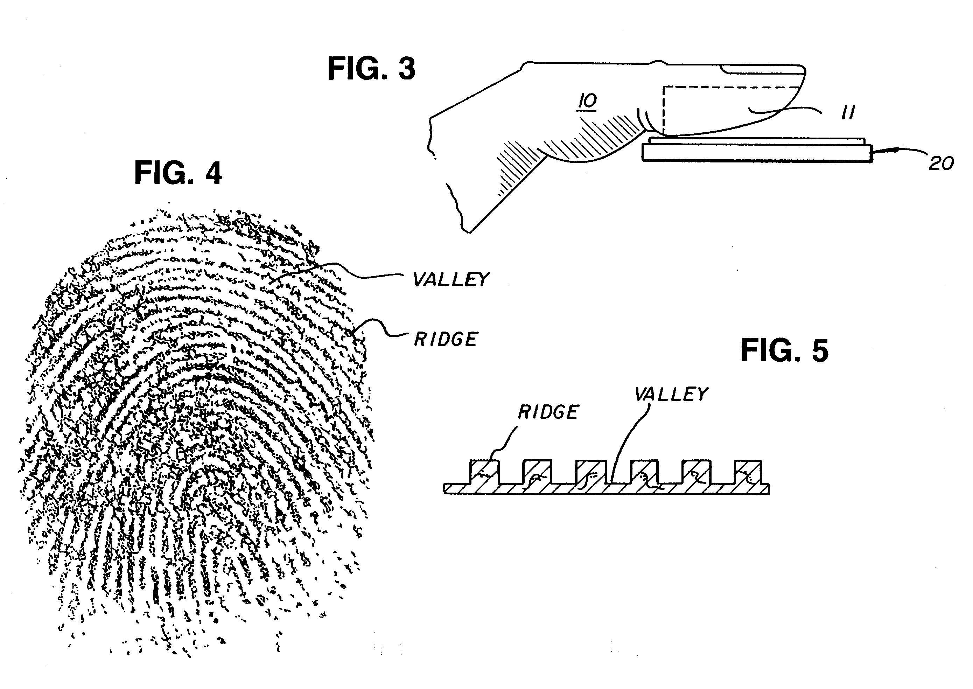

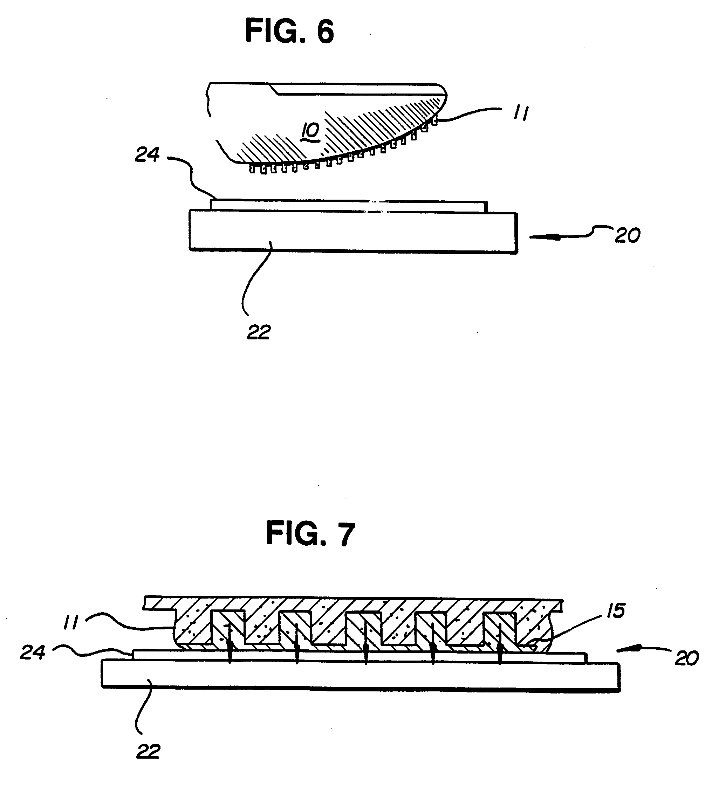

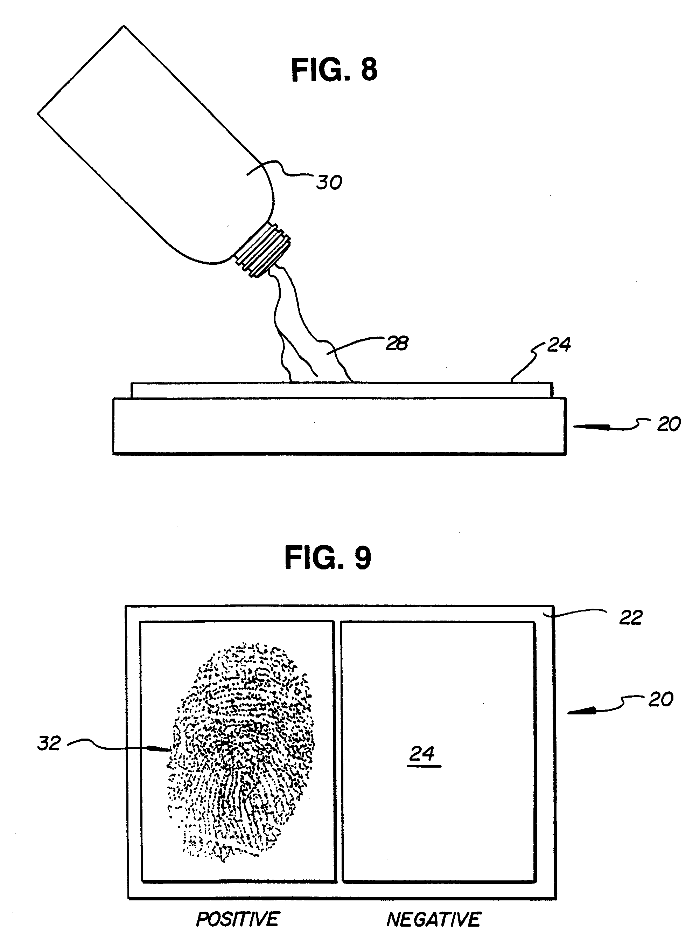

The preferred embodiment and best mode of the invention is shown in Figures 1-9. In the invention, a finger 10 with swirls or ridges 11 is pricked with a needle 12 to obtain a blood droplet 14 as is shown in Figure 2. A spatula 16 spreads the thin film of blood over the finger 10 and the fingertip is then applied to testing pad 20 comprised of absorbent pad 22 upon which is mounted a membrane 24 impregnated with specified antibody or antigen which will capture specific antigen or antibodies present in the human body such as a drug, biological agent or substance which has been taken by the individual. A schematic cross section is shown in Figure 7 of the finger showing a thin film of blood 15 on top of the ridges 11 which will have a low concentration of antigen and a thicker amount of blood 14 between the ridges 11 which will have a higher concentration of antigen. Thus when the membrane is incubated with a chromogenic substrate a reverse fingerprint will be developed. The membrane 24 is then incubated with a chromogenic substrate 28 as shown carried in bottle 30 and a color 32 is developed whose intensity is proportional to the antigen or antibody present.

The chromogenic substrate provides a sensitive detection method for the antigen or antibody. Generally, the color produced is proportional to the amount of unknown in the sample, providing the unknown is the limiting component of the system. The BCIP,NBT Phosphates Substrate System generates a dark purple stain on membrane sites bearing phosphatase. Alkaline phosphatase catalyze the dephosphorylation of 5-bromo-4-chloro-3 indolyl phosphate which initiates a reaction cascade resulting in intense color formation.

Binding of an antibody can be detected by a variety of reagent systems as is the case for antigen bound to the antibodies on the membrane. For instance, I-labeled antimouse immunoglobulin or I-labeled protein A may be used. Antimouse immunoglobulin conjugated directly to alkaline phosphatase or to peroxidase may be used, together with appropriate chromogenic substrates. The biotin-avidin peroxidase system can be used together with appropriate chromogenic substrates. The biotin-avidin peroxidase system (for example, the Vectastain ABC system supplied by Vector Laboratories) is particularly sensitive.

The solid phase membranes eliminate handling, allow the product configuration to be cut in the desired shape or format, and provides faster kinetics and increased protein binding. Protein binding to solid plastic substrates Has been found to be a non-stoichiometric process and varies greatly by the type of plastic used. Binding is not specific and generally occurs through electrostatic and hydrophobic interreactions between plastic and proteins. Membrane substrates overcome many of the problems inherent in solid phase immunoassays as they combine the qualities of a solid substrate with a range of expanded capabilities and, due to their porosity and consequential large surface area, have a high protein binding capacity. A protein binding capacity is increased by using smaller pore sized membranes whose total binding surface increases for an equivalent frontal surface.

Membranes which can be used in the present invention can be constructed of nitrocellulose, nylon, cellulose or Immuno Affinity Membranes (IAM) produced by Millipore Inc. The choice of adsorbing matrix depends on the physical properties such as sensitivity, binding capacity, stability or bound molecules and compatibility with the assay system.

The prefered membrane used is Nylon because the finger print color is retained and not washed away when the chromogenic substrate alkaline phosphatase is added to positively test for the specific antibody or antigen being tested for. Membranes, such as nylon and cellulose, can be modified to create surface sites for covalent binding of proteins. Nitrocellulose is one of the most commonly used membranes due to its high affinity for proteins and cellular macromolecules. In IAM, polyvinylidenedifluoride (PVDF), the base polymer of IAM is hydrophobic and binds proteins. IAM permits a high degree of control over the extent of protein binding and the user can reproducibly immobilize nanogram to microgram quantities of protein on the surface to suit various assay requirements. Binding the protein to IAM surfaces occurs primarily though the epsilon amino group of lysine, which contrasts the binding proteins to nitrocellulose, nylon or plastic where the bonding is ionic or hydrophobic.

In one experiment, conducted by Millipore Corp. radiolabeled IgG was bound to IAM, nitrocellulose (SWAP, Millipore Corp.), a nylon-based affinity membrane (BIODYNE IMMUNO AFFINITY MEMBRANE. Pall Inc.), and nylon (NYTRAN, Schleicher and Schuell). The membranes were then immersed in serum and the protein retained on the membrane was measured in intervals over a period of 20 hr. After 1 hr incubation, less than 15% of the original protein (which includes the unbound protein in the interstitial volume) has eluted from the IAM, while over 40% of the protein has desorbed from the nylon-based affinity membrane. The nylon and nitrocellulose membranes retained less than half of the originally bound protein after 1 hr of incubation. Thus the loss of IgG from IAM after 1hr is small, while the other membranes continue to lose protein, presumably due to exchange. However, because the present test is done within a short period of time such retention problems are not significant.

Another type of membrane which can be used in the invention which has previously been noted is nitrocellulose which provides an excellent matrix for blotting proteins and nucleic acids. The nitrocellulose may be cut into whatever shape is required and has the useful characteristic that the amount of color produced by the fingerprint will be clearly visible. Pure nitrocellulose adsorbs proteins, nucleic acids and other cellular antigens. These adsorbed substances often retain antigen-antibody binding activity and can be visualized using ultrasensitive, enzyme amplified immunostaining methods so that a chromogenic stain marks the location of the adsorbed materials. This approach uses a technique called Dot ELISA, (which also can be utilized with the Nylon, IAM, plastic membranes) whereby nanogram amounts of protein are directly applied to nitrocellulose. One important advantage of Dot ELISA is the ability to perform multiple enzyme immunoassays in a single test procedure using as little as one microliter of antigen or capture antibody solution. Nanogram amounts of capture antibodies dotted onto a single membrane can be used to screen simultaneously for a variety of antigens. In a Dot ELISA procedure the reactant is diluted in coating solution and dotted onto the damp membrane. While the optimal concentration will vary from reactant to reactant, for complex antigens 0.1-1.0 mg/ml is suitable. Following membrane blotting excess binding sites are blocked by thoroughly soaking both sides of the membrane in Diluent/Blocking Solution. Any of a variety of reservoirs can be used. The Diluent/Blocking Solution contains 1% bovine serum albumin (BSA) in phosphate buffered saline which protects adsorbed protein from surface denaturation. Following the blocking step, membranes can be stored dry at refrigeration temperatures for several months without loss of activity. The adsorption of an antigen or capture antibody onto the nitrocellulose membrane can be accomplished by Antigen Detection ELISA, which is the simplest method for detection of antigen, Indirect Antibody ELISA which is capable of detecting either antibody or antigen, depending on which is defined as the unknown or Antibody Sandwich ELISA which is accomplished by adsorption or an antigen or capture antibody, washing each reagent of any free or unattached reactant and adding another reagent to build step by step a molecular sandwich on the membrane surface which is completed by the addition of an enzyme-antibody conjugate. The construction of such membrane surfaces is clearly shown by a bulletin of Kirkegaard & Perry Laboratories, Inc. 1985 entitled ELISAmate (TM) Enzyme Immunoassay Test System for Detection of Antigens or Antibodies on Membranes.

Alternately a secondary antibody can be mixed with the blood and complexes with the antigen in the blood. This secondary antibody is provided with color label which colors the fingerprint in negative or reverse order of a normal print in that the valleys contain a majority of the antigen or antibody and therefore antigen labelled antibody complexes so that the coloration reacts most strongly with the valleys defined by the ridges with the ridges of the fingerprinting being light rather than the dark coloration. The enzyme in the conjugate serves as an indicator that upon reaction with substrate demonstrates the presence of unknown in the sample. The chromogenic substrate provides a detection for the conjugate enzyme and the color produced is proportional to the amount of the unknown in the sample providing the unknown is the limiting component of the system. The antibodies can be labelled with HRP (horseradish peroxidase), an enzyme that detoxifies hydrogen peroxide, H₂O₂, by converting it to water. HRP initiates this transformation when it gives hydrogen peroxide a pair of electrons. The enzyme subsequently collects these electrons from suitable donors. Thus the total color generated by peroxidase depends upon the relative rates of color generation and product inactivation of the enzyme.

The color solution used is a substrate manufactured by Kirkegaard & Perry Labs under one of several acronyms namely: ABTS (2, 2'-azino-di-[3-ethylbenzthiazoline sulfonate (6)]; OPD (orthophenylene diamine); or TMB (tetramethylbenzidine). In choosing the substrate, the sensitivity of the immunoassay is determined by the discrimination of the antibody reagents. When this occurs, the use of a more sensitive substrate serves only to proportionately increase the signal and the background.

If the assay optimization indicates the sensitivity of the immunoassay is limited by the color generated by the substrate then the more sensitive substrate would give more color development without a corresponding increase in the background.

If desired, protein solutions such as bovine serum albumin (3%), fetal calf serum (1 to 5%) or gelatin (1%), or mixtures of these proteins may be used to block nonspecific binding of antibody to the nitrocellulose. The efficacy of blocking is assessed on the basis of the background staining intensity.

## Claims

1. A test device, for testing for the presence of at least one substance in a fluid sample obtained from a human being whilst simultaneously positively identifying the sample as coming from the test subject, comprising a porous or permeable membrane (24) mounted to or over an absorbent pad (22), said porous or permeable membrane (24) comprising at least one immobilized first antibody or antigen to capture a concentrated amount of at least one antigen or antibody specific to said at least one first antigen or antibody from the fluid sample in direct proportion to the concentration of the at least one antigen or antibody in the fluid sample, said porous or permeable membrane (24) being simultaneously capable of retaining a fingerprint imprint from the test subject's finger.

2. A test device as claimed in claim 1, wherein said porous or permeable membrane comprises an immobilised first antibody to capture a concentrated amount of an antigen specific to said first antibody.

3. A test device as claimed in claim 1 wherein said porous or permeable membrane comprises a plurality of different immobilised first antibodies to capture a concentrated amount of a plurality of different antigens specific to said first antibodies.

4. A test device as claimed in claim 1 wherein said porous or permeable membrane comprises an immobilised first antigen to capture a concentrated amount of an antibody specific to said first antigens.

5. A test device as claimed in claim 1, wherein said porous or permeable membrane comprises a plurality of different immobilised first antigens to capture a concentrated amount of a plurality of different antibodies specific to said first antigens.

6. A test device as claimed in claim 3 or 5, in which the porous or permeable membrane comprises a plurality of separated areas each provided with a different immobilised first antibody or antigen to capture a concentrated amount of a different antigen or antibody specific to said different first antibody or antigen.

7. A test device as claimed in any of the previous claims wherein the porous or permeable membrane is a nitrocellulose membrane.

8. A test device as claimed in any of the previous claims which tests for the presence of at least one drug.

9. A test device as claimed in claim 4 or 5 wherein the immobilised first antigen/antigens is/are specific to an antibody/antibodies produced in response to a steroid, a steroid metabolite or a by-product thereof.

10. A test device as claimed in claim 4 or 5 wherein the immobilised first antigen/antigens is/are specific to an antibody/antibodies produced in response to cocaine, heroin or a metabolite thereof.

11. A test device as claimed in claim 4 or 5 wherein the immobilised first antigen/antigens is/are specific to an antibody/antibodies produced in response to marijuana or an amphetamine.

12. A test device as claimed in any of the previous claims wherein the fluid sample is blood.

13. A method, for testing for a substance in a fluid sample obtained from a human being whilst simultaneously positively identifying the sample as coming from the test subject, comprising the steps of:
a) obtaining a fluid sample from the test subject;
b) covering the finger with a thin film of the fluid sample;
c) placing the finger on a porous or permeable membrane (24) having an absorbent pad mounted thereto or behind so that the fluid is deposited from the finger onto the porous or permeable membrane (24), said porous or permeable membrane (24) comprising at least one immobilised first antibody or antigen to capture a concentrated amount of at least one antigen or antibody specific to said first antigen or antibody from the fluid sample in direct proportion to the concentration of the at least one antigen or antibody specific to said first antigen or antibody in the fluid sample; and
d) adding a colour development solution to the porous or permeable membrane (24) to identify the presence or absence of said at least one substance and identify the test subject's fingerprint.

14. A method as claimed in claim 13 wherein the fluid obtained is blood.

15. A method as claimed in claim 14 wherein the finger of the test subject is pricked to obtain a quantity of blood on the finger.

16. A method of testing as claimed in any of claims 13 to 15, wherein the immobilised first antigen/antigens is/are specific to an antibody/antibodies produced in response to a steroid, a steroid metabolite or a by-product thereof.

17. A method of testing as claimed in any of claims 13 to 15 wherein the immobilised first antigen/antigens is/are specific to an antibody/antibodies produced in response to cocaine, heroin or a metabolite thereof.

18. A method of testing as claimed in any of claims 13 to 15, wherein the immobilised first antigen/antigens is/are specific to an antibody/antibodies produced in response to marijuana or an amphetamine.

## Patentansprüche

1. Testvorrichtung zum Testen auf das Vorliegen von mindestens einer Substanz in einer flüssigen Probe, die von einem Menschen gewonnen wurde, während gleichzeitig positiv identifiziert wird, daß die Probe von der Testperson stammt, die folgendes umfaßt: Eine poröse oder permeable Membran (24), die auf einem oder über einem saugfähigen Kissen (22) befestigt ist, wobei genannte poröse oder permeable Membran (24) mindestens einen immobilisierten ersten Antikörper oder ein immobilisiertes erstes Antigen umfaßt, um eine konzentrierte Menge von mindestens einem Antigen oder Antikörper festzuhalten, das/der für genanntes mindestens eine erste Antigen oder genannten mindestens einen ersten Antikörper aus der flüssigen Probe spezifisch ist, in direkter Proportionalität zu der Konzentration von dem mindestens einen Antigen oder Antikörper in der flüssigen Probe, wobei genannte poröse oder permeable Membran (24) gleichzeitig dazu in der Lage ist, einen Fingerprint-Abdruck von dem Finger der Testperson zurückzubehalten.

2. Testvorrichtung nach Anspruch 1, worin genannte poröse oder permeable Membran einen immobilisierten ersten Antikörper umfaßt, um eine konzentrierte Menge eines Antigens festzuhalten, das für genannten ersten Antikörper spezifisch ist.

3. Testvorrichtung nach Anspruch 1, worin genannte poröse oder permeable Membran eine Vielzahl verschiedener immobilisierter erster Antikörper zum Festhalten einer konzentrierten Menge einer Vielzahl verschiedener Antigene umfaßt, die für genannte erste Antikörper spezifisch sind.

4. Testvorrichtung nach Anspruch 1, worin genannte poröse oder permeable Membran ein immobilisiertes erstes Antigen zum Festhalten einer konzentrierten Menge eines Antikörpers umfaßt, der für genannte erste Antigene spezifisch ist.

5. Testvorrichtung nach Anspruch 1, worin genannte poröse oder permeable Membran eine Vielzahl verschiedener immobilisierter erster Antigene zum Festhalten einer konzentrierten Menge einer Vielzahl verschiedener Antikörper umfaßt, die für genannte erste Antigene spezifisch sind.

6. Testvorrichtung nach Anspruch 3 oder 5, worin die poröse oder permeable Membran eine Vielzahl getrennter Bereiche umfaßt, wobei jeder mit einem verschiedenen immobilisierten ersten Antikörper oder Antigen zum Festhalten einer konzentrierten Menge eines verschiedenen Antigens oder Antikörpers vorgesehen ist, das/der für genannten verschiedenen ersten Antikörper oder genanntes verschiedenes erstes Antigen spezifisch ist.

7. Testvorrichtung nach einem der vorangehenden Ansprüche, worin die poröse oder permeable Membran eine Nitrocellulosemembran ist.

8. Testvorrichtung nach einem der vorangehenden Ansprüche, die auf das Vorliegen von mindestens einer Droge testet.

9. Testvorrichtung nach Anspruch 4 oder 5, worin das/die immobilisierte(n) erste(n) Antigen/Antigene für einen Antikörper/für Antikörper spezifisch ist/sind, der/die als Antwort auf ein Steroid, einen Steroidmetaboliten oder ein Nebenprodukt davon spezifisch ist/sind.

10. Testvorrichtung nach Anspruch 4 oder 5, worin das/die immobilisierte(n) erste(n) Antigen/Antigene für einen Antikörper/für Antikörper spezifisch ist/sind, der/die als Antwort auf Cocain, Heroin oder einen Metaboliten davon produziert wird/werden.

11. Testvorrichtung nach Anspruch 4 oder 5, worin das/die immobilisierte(n) erste(n) Antigen/Antigene für einen Antikörper/für Antikörper spezifisch ist/sind, der/die als Antwort auf Marihuana oder ein Ampethamin produziert wird/werden.

12. Testvorrichtung nach einem der vorangehenden Ansprüche, worin die flüssige Probe Blut ist.

13. Verfahren zum Testen auf eine Substanz in einer flüssigen Probe, die von einem Menschen gewonnen wird, während die Probe gleichzeitig positiv als von der Testperson kommend identifiziert wird, welches die folgenden Schritte umfaßt:
a) Gewinnen einer flüssigen Probe von der Testperson;
b) Abdecken des Fingers mit einem dünnen Film der flüssigen Probe;
c) Auflegen des Fingers auf eine poröse oder permeable Membran (24), die ein saugfähiges Kissen darauf oder dahinter befestigt hat, damit die Flüssigkeit von dem Finger auf die poröse oder permeable Membran (24) abgelagert wird, wobei genannte poröse oder permeable Membran (24) mindestens einen immobilisierten ersten Antikörper oder ein immobilisiertes erstes Antigen zum Festhalten einer konzentrierten Menge von mindestens einem Antigen oder Antikörper umfaßt, das/der für genanntes erstes Antigen oder genannten ersten Antikörper aus der flüssigen Probe spezifisch ist, in direkter Proportionalität zu der Konzentration des mindestens einen Antigens oder Antikörpers, das/der für genanntes erstes Antigen oder genannten ersten Antikörper in der flüssigen Probe spezifisch ist und
d) Zufügen einer Farbentwicklungslösung zu der porösen oder permeablen Membran (24), um die Anwesenheit oder Abwesenheit von genannter mindestens einer Substanz zu identifizieren und um den Fingerprint der Testperson zu identifizieren.

14. Verfahren nach Anspruch 13, worin die gewonnene Flüssigkeit Blut ist.

15. Verfahren nach Anspruch 14, worin zum Gewinnen einer Blutmenge auf dem Finger in den Finger der Testperson gestochen wird.

16. Verfahren zum Testen nach einem der Ansprüche 13 bis 15, worin das/die immobilisierte(n) erste(n) Antigen/ Antigene für einen Antikörper/für Antikörper spezifisch ist/sind, der/die als Antwort auf ein Steroid, einen Steroidmetaboliten oder ein Nebenprodukt davon produziert wird/werden.

17. Verfahren zum Testen nach einem der Ansprüche 13 bis 15, worin das/die immobilisierte(n) erste(n) Antigen/Antigene für einen Antikörper/für Antikörper spezifisch ist/sind, der/die als Antwort auf Cocain, Heroin oder einen Metaboliten davon produziert wird/werden.

18. Verfahren zum Testen nach einem der Ansprüche 13 bis 15, worin das/die immobilisierte(n) erste(n) Antigen/Antigene für einen Antikörper/für Antikörper spezifisch ist/sind, der/die als Antwort auf Marihuana oder ein Amphetamin produziert wird/werden.

## Revendications

1. Dispositif d'analyse, pour analyser la présence d'au moins une substance dans un échantillon de fluide obtenu d'une personne tout en identifiant simultanément de manière sûre l'échantillon comme provenant du sujet testé, comprenant une membrane poreuse ou perméable (24) montée sur ou par dessus un tampon absorbant (22), ladite membrane poreuse ou perméable (24) comprenant au moins un premier anticorps ou antigène immobilisé pour capter une quantité concentrée d'au moins un antigène ou anticorps spécifique vis-à-vis dudit au moins un premier antigène ou anticorps provenant de l'échantillon de fluide en proportion directe de la concentration dudit au moins un antigène ou anticorps dans l'échantillon de fluide, ladite membrane poreuse ou perméable (24) étant simultanément capable de retenir une empreinte digitale du doigt du sujet testé.

2. Dispositif d'analyse selon la revendication 1, dans lequel ladite membrane poreuse ou perméable comprend un premier anticorps immobilisé pour capter une quantité concentrée d'un antigène spécifique vis-à-vis dudit premier anticorps.

3. Dispositif d'analyse selon la revendication 1, dans lequel ladite membrane poreuse ou perméable comprend une pluralité de premiers anticorps immobilisés différents pour capter une quantité concentrée d'une pluralité d'antigènes différents spécifiques vis-à-vis desdits premiers anticorps.

4. Dispositif d'analyse selon la revendication 1, dans lequel ladite membrane poreuse ou perméable comprend un premier antigène immobilisé pour capter une quantité concentrée d'un anticorps spécifique vis-à-vis dudit premier antigène.

5. Dispositif d'analyse selon la revendication 1, dans lequel ladite membrane poreuse ou perméable comprend une pluralité de premiers antigènes immobilisés différents pour capter une quantité concentrée d'une pluralité d'anticorps différents spécifiques vis-à-vis desdits premiers antigènes.

6. Dispositif d'analyse selon la revendication 3 ou 5, dans lequel la membrane poreuse ou perméable comprend une pluralité de zones séparées dont chacune est pourvue d'un premier anticorps ou antigène immobilisé différents pour capter une quantité concentrée d'antigène ou d'anticorps différents spécifiques vis-à-vis desdits premiers anticorps ou antigène différents.

7. Dispositif d'analyse selon l'une quelconque des revendications précédentes, dans lequel la membrane poreuse ou perméable est une membrane en nitrocellulose.

8. Dispositif d'analyse selon l'une quelconque des revendications précédentes, qui analyse la présence d'au moins une drogue.

9. Dispositif d'analyse selon la revendication 4 ou 5, dans lequel le(s) premier(s) antigène (s) immobilisé(s) est(sont) spécifique(s) vis-à-vis d'un ou de plusieurs anticorps produit(s) en réponse à un stéroïde, un métabolite de stéroïde ou un sous-produit de ceux-ci.

10. Dispositif d'analyse selon la revendication 4 ou 5, dans lequel le(s) premier (s) antigène (s) immobilisé(s) est (sont) spécifique(s) vis-à-vis d'un ou de plusieurs anticorps produit(s) en réponse à la cocaïne, l'héroïne ou un métabolite de celles-ci.

11. Dispositif d'analyse selon la revendication 4 ou 5, dans lequel le(s) premier(s) antigène(s) immobilisé(s) est(sont) spécifique(s) vis-à-vis d'un ou de plusieurs anticorps produit(s) en réponse à la marijuana ou une amphétamine.

12. Dispositif d'analyse selon l'une quelconque des revendications précédentes, dans lequel l'échantillon de fluide est du sang.

13. Procédé pour analyser la présence d'une substance dans un échantillon de fluide obtenu d'une personne tout en identifiant simultanément de façon sûre l'échantillon comme provenant du sujet testé, comprenant les étapes consistant à :
a) obtenir un échantillon de fluide du sujet testé ;
b) recouvrir le doigt avec un film mince de l'échantillon de fluide ;
c) placer le doigt sur une membrane poreuse ou perméable (24) sur ou derrière laquelle est monté-un tampon absorbant, de manière à ce que le fluide se dépose du doigt sur la membrane poreuse ou perméable (24), ladite membrane poreuse ou perméable (24) comprenant au moins un premier anticorps ou antigène immobilisé pour capter une quantité concentrée d'au moins un antigène ou anticorps spécifique vis-à-vis dudit premier antigène ou anticorps provenant de l'échantillon de fluide en proportion directe de la concentration dudit au moins un antigène ou anticorps spécifique vis-à-vis dudit premier antigène ou anticorps dans l'échantillon de fluide ; et
d) ajouter une solution de développement de couleur à la membrane poreuse ou perméable (24) pour identifier la présence ou l'absence de ladite au moins une substance et identifier l'empreinte digitale du sujet testé.

14. Procédé selon la revendication 13, dans lequel le fluide obtenu est du sang.

15. Procédé selon la revendication 14, dans lequel le doigt du sujet testé est piqué pour obtenir une quantité de sang sur le doigt.

16. Procédé d'analyse selon l'une quelconque des revendications 13 à 15, dans lequel le(s) premier(s) antigène (s) immobilisé(s) est (sont) spécifique (s) vis-à-vis d'un ou plusieurs anticorps produit(s) en réponse à un stéroïde, un métabolite de stéroïde ou un sous-produit de ceux-ci.

17. Procédé d'analyse selon l'une quelconque des revendications 13 à 15, dans lequel le(s) premier(s) antigène (s) immobilisé(s) est (sont) spécifique(s) vis-à-vis d'un ou plusieurs anticorps produit(s) en réponse à la cocaïne, l'héroïne ou un métabolite de celles-ci.

18. Procédé d'analyse selon l'une quelconque des revendications 13 à 15, dans lequel le(s) premier(s) antigène(s) immobilisé(s) est(sont) spécifique(s) vis-à-vis d'un ou plusieurs anticorps produit(s) en réponse à la marijuana ou une amphétamine.
